# EUROPEAN PATENT SPECIFICATION

(11) **EP 2 765 203 B1**
(45) Date of publication and mention of the grant of the patent: **04.07.2018**
(21) Application number: 14151082.6
(22) Date of filing: 14.01.2014
(51) Int. Cl.: C12Q 1/68

(54) **BLOOD COLLECTION DEVICE FOR STABILIZING CELL-FREE RNA IN BLOOD DURING SAMPLE SHIPPING AND STORAGE**
BLUTENTNAHMEVORRICHTUNG ZUR STABILISIERUNG VON ZELLFREIER RNA BEI BLUTPROBENTRANSPORT UND -LAGERUNG
DISPOSITIF DE PRÉLÈVEMENT SANGUIN POUR LA STABILISATION D'ARN ACELLULAIRE DANS LE SANG LORS DU TRANSPORT ET DU STOCKAGE D'ÉCHANTILLON

(30) Priority: 14.01.2013 US 201361751983 P
(43) Date of publication of application: 13.08.2014
(73) Proprietor: Streck Inc., La Vista, NE 68128 (US)
(72) Inventor: Ryan, Wayne L., Omaha, NE 68130 (US); Fernando, Rohan, Omaha, NE 68136 (US); Qin, Jianbing, Omaha, NE 68135 (US)
(74) Representative: Bawden, Peter Charles

(56) References cited:
- WO-A1-2010/096323
- WO-A1-2011/057184
- M. ROHAN FERNANDO ET AL: "Stabilization of cell-free RNA in blood samples using a new collection device", CLINICAL BIOCHEMISTRY, vol. 45, no. 16-17, 1 November 2012 (2012-11-01), pages 1497-1502, XP055118091, ISSN: 0009-9120, DOI: 10.1016/j.clinbiochem.2012.07.090
- M R Fernando ET AL: "Stabilization of Cell-Free RNA in Plasma for Noninvasive Diagnosis and Prognosis", , 5 August 2010 (2010-08-05), XP055118118, Retrieved from the Internet: URL:http://www.streck.com/resources/Cell_S tabilization/Cell-Free_RNA_BCT/02_Product_ Information/02_Paper_Cell-Free_RNA_BCT_Sta bilization_of_Cell-Free_RNA_in_Plasma.pdf [retrieved on 2014-05-15]

## Description

### FIELD OF THE INVENTION

This invention relates to a method for improved protection and regulation of nucleic acid materials during collection, storage, and shipment.

### BACKGROUND OF THE INVENTION.

Cell-free RNA (cfRNA) naturally occurs in blood and has the potential to be used for non-invasive prenatal diagnostics and for detection, monitoring, and molecular analysis of biomarkers for cancer and other diseases. Since cfRNA targets are present in blood at low quantities, it is important to minimize release of cellular RNA in a blood sample following blood draw. Pre-analytical conditions can affect the release of background (e.g., cellular) RNA into plasma, decreasing the proportion of specific cfRNA targets and masking their detection in downstream applications (e.g., polymerase chain reaction, flow cytometric and other analysis protocols). Due to the low abundance of the cfRNA biomarkers, it is recommended that genomic RNA background levels be minimized to provide accurate measurements cfRNA levels. Therefore, it is necessary to address several pre-analytical issues that arise during the time between blood draw and RNA isolation. These include delays in blood processing, blood storage temperature, and agitation of the sample during transport and shipment of blood. Such conditions may alter plasma RNA levels by causing cellular RNA release from blood cells that obfuscate true cfRNA. There is thus a clear need for protocols that stabilize cfRNA in blood as well as maintain cfRNA integrity during sample processing and shipping. Fernando et al., 2012 (Clinical Biochemistry) discloses the stabilisation of cell-free RNA in blood samples. However, this publication does not disclose the chemical composition used for stabilising the samples.

### SUMMARY OF THE INVENTION

The cell-free RNA collection devices disclosed herein prevent increases in background RNA levels caused by temperature fluctuations or agitation that can occur during blood sample storage and shipping. These blood collection devices provide a method for obtaining high quality stabilized cfRNA samples for rare RNA target detection and determining accurate cfRNA concentrations.

In one aspect, the present teachings contemplate a method for handling a biological sample to a remote site for cell-free RNA analysis, comprising the steps of: drawing a blood sample directly into a stabilizing blood collection device at a blood draw site, such blood collection device including an effective amount of a stabilizing agent selected from imidazolidinyl urea and glycine and diazolidinyl urea and glycine, the sample of blood including an initial amount of background RNA and an amount of cell free RNA; treating one or more components of the sample with the stabilizing agent for mitigating the propensity of background RNA within the sample to increase relative to the initial amount; transporting the sample, while it remains in the blood collection device, for delivery to site at which cell-free RNA analysis is to be performed, during which transporting step the blood collection device and the sample contained therein is subjected to one or more temperatures within a range of 5 to 35 °C; performing cell free RNA analysis on the sample at least 24 hours after the drawing of the sample..

The sample of blood includes an initial amount of background RNA and an amount of cell free RNA. The method includes the step of handling the sample, while it remains in the blood collection device, for delivery to a site at which cell-free RNA analysis is to be performed, during which transporting step the blood collection device and the sample contained therein is subjected to one or more temperatures within a range of about 5 to about 35°C. The method includes performing analysis on the cell free RNA from within the sample.

The stabilizing blood collection device includes imidazolidinyl urea and glycine or diazolidinyl urea and glycine. The stabilizing blood collection device may also include aurintricarboxylic acid and sodium fluoride. The stabilizing blood collection device may include some combination of diazolidinyl urea, imidazolidinyl urea, aurintricarboxylic acid, glyceraldehyde, and sodium fluoride, and EDTA.

During the step of sample handling (which includes but is not limited to sample transport), the sample may be subjected to a temperature below room temperature (e.g., below about 15°C, 10°C, or even 7°C) throughout at least a portion (e.g., at least one tenth, one quarter, or one half) of the duration of the handling step. During the step of sample handling the sample may be subjected to a temperature above room temperature (e.g., above about 25°C or even 30°C) throughout at least a portion (e.g., at least one tenth, one quarter, or one half) of the duration of the handling step. During the step of sample handling the sample may be subjected to irregular and/or uncontrolled periods of vibration and/or agitation. The duration of the handling step is for at least about 24 hours. The duration of the handling step may be for at least about 72 hours. The step of performing cell free RNA analysis on the sample may include performing transcriptase real-time PCR (RT-qPCR). The step of performing cell free RNA analysis on the sample may include performing transcriptase real-time PCR (RT-qPCR) to quantify mRNAs for c-fos, β-actin, and/or 18S rRNA. The sample treated with the stabilizing agent may exhibit an increase in mRNA copy numbers that is less than 50% that of a sample handled in the absence of the treating step. The handling step may include transporting the sample from a blood draw site to a site for cell free RNA analysis in a transportation vehicle (e.g., selected from a truck, a train, an airplane, a helicopter, an automobile, a watercraft or the like).

The teachings herein further contemplate that the methods disclosed herein result in the cellular mRNA copy number per mL of plasma within the blood sample remaining substantially the same prior to shipping and post-shipping. The teachings herein further contemplate that the methods disclosed herein result in the c-fos mRNA copy number per mL of plasma within the blood sample remaining substantially the same prior to shipping and post-shipping. The β-Actin mRNA copy number per mL of plasma within the blood sample may remain substantially the same prior to shipping and post-shipping. The 18s rRNA copy number per mL of plasma within the blood sample may remain substantially the same prior to shipping and post-shipping. The amount of background RNA in the blood sample may remain substantially the same prior to shipping and post shipping. The c-fos mRNA copy number per mL of plasma within the blood sample may remain substantially the same when stored at 6°C, 22°C, and 30°C for 3 days. The β-Actin mRNA copy number per mL of plasma within the blood sample may remain substantially the same when stored at 6°C, 22°C, and 30°C for 3 days. The amount of background RNA in the blood sample may remain substantially the same when stored at 6°C, 22°C, and 30°C for 3 days.

### BRIEF DECSRIPTION OF THE DRAWINGS

**Figure 1** - **Shipping temperature record** - showing the temperature of selected blood samples over time during shipment.
**Figure 2A** - **C-fos mRNA copy number per milliliter of plasma** - showing the mRNA copy number (using c-fos markers) in initial, not shipped and shipped blood samples contacted with EDTA and the same for blood samples located in the stabilizing blood collection devices taught herein.
**Figure 2B** - **β-Actin mRNA copy number per milliliter of plasma** - showing the mRNA copy number (using β-Actin markers) in initial, not shipped and shipped blood samples contacted with EDTA and the same for blood samples located in the stabilizing blood collection devices taught herein.
**Figure 2C** - **18s rRNA copy number per milliliter of plasma** - showing the rRNA copy number (using 18s rRNA markers) in initial, not shipped and shipped blood samples contacted with EDTA and the same for blood samples located in the stabilizing blood collection devices taught herein.
**Figure 3A** - **C-fos mRNA copy number per milliliter of plasma** - showing the mRNA copy number (using C-fos mRNA markers) at initial draw, 6°C storage temperature, 22°C storage temperature and 30°C storage temperature blood samples contacted with EDTA and the same for blood samples located in the stabilizing blood collection devices taught herein.
**Figure 3B** - **β-Actin mRNA copy number per milliliter of plasma** - showing the mRNA copy number (using β-Actin mRNA markers) at initial draw, 6°C storage temperature, 22°C storage temperature and 30°C storage temperature blood samples contacted with EDTA and the same for blood samples located in the stabilizing blood collection devices taught herein.

### DETAILED DESCRIPTION

This application claims the benefit of the priority date of United States Provisional Application Serial No. 61/751,983, filed January 14, 2013. This application is also related to U.S. Patent No. 8,304,187, filed on February 11, 2010.

The explanations and illustrations presented herein are intended to acquaint others skilled in the art with the teachings, its principles, and its practical application. Those skilled in the art may adapt and apply the teachings in its numerous forms, as may be best suited to the requirements of a particular use. Accordingly, the specific embodiments of the present teachings as set forth are not intended as being exhaustive or limiting of the teachings. The scope of the teachings should, therefore, be determined not with reference to the above description, but should instead be determined with reference to the appended claims, along with the full scope of equivalents to which such claims are entitled.

Recent studies have shown blood plasma to contain low abundance cfRNA targets, an example of which is circulating tumor cell-derived RNA. Polymerase chain reaction detection of these cell-free targets within a high amount of cellular background RNA is challenging, requiring specialized protocols and/or large volumes of starting material. As a result, minimizing the release of cellular RNA from nucleated cells (e.g., background RNA) is essential for accurate analysis of true cfRNA.

In current practice, it is recommended that blood samples be immediately centrifuged to isolate and freeze plasma to prevent background RNA contamination of cfRNA during sample processing, transportation and storage. However, the examples discussed herein demonstrate that the stabilizing blood collection method of the present invention prevents the release of background RNA into plasma post-phlebotomy for up to 3 days, thus avoiding these labor-intensive requirements (e.g., centrifugation and freezing). Using the stabilizing blood collection method disclosed herein, *ex vivo* storage at room temperature becomes possible, allowing flexibility for offsite blood draws to be sent to central laboratories for downstream analysis of the cfRNA without preliminary centrifugations or cryopreservation.

As mentioned above, transportation of blood samples from the site of phlebotomy to another facility is commonly required for molecular diagnostic testing. In this regard, several studies have focused on pre-analytical variables that might compromise the accuracy cfRNA measurements, including the selection of blood collection devices, sample storage and shipping conditions. Each of these parameters affects the amount of nucleated blood cell lysis that occurs post-phlebotomy. Such nucleated cell lysis leads to release of cellular RNA, elevating RNA backgrounds and suppressing true and accurate cfRNA measurement. Accordingly, inaccurate cfRNA measurement reduces or even eliminates the utility of such measurements for disease diagnosis, genetic testing or other purposes. The method disclosed herein allow for minimized background RNA increases by utilizing a stabilizing blood collection method with the ability to stabilize nucleated blood cells during shipping movement and temperature fluctuations. The examples discussed herein evaluate the ability of the stabilizing blood collection method taught herein and traditional K₃EDTA tubes to preserve cfRNA and prevent background RNA release when subjected to conditions that can occur during sample storage and shipping.

During transport of samples, shaking may disrupt nucleated blood cell integrity and compromise accuracy of sample testing, as described above. For the examples described herein, blood samples were shipped in either K₃EDTA tubes or the stabilizing blood collection device taught herein. The resulting mRNA or rRNA copy numbers (as measured by c-fos, β-Actin or 18s RNA markers) showed increased background RNA for the shipped samples in the K₃EDTA tubes as compared to those samples in the stabilizing blood collection device taught herein. The stabilizing blood collection device samples showed stable mRNA or rRNA copy numbers before and after shipping. This suggests nucleated cell disruption occurred in blood samples that were shipped in K₃EDTA leading to cellular RNA release, as this did not occur in the samples subjected to the method of this invention within the stabilizing blood collection device.

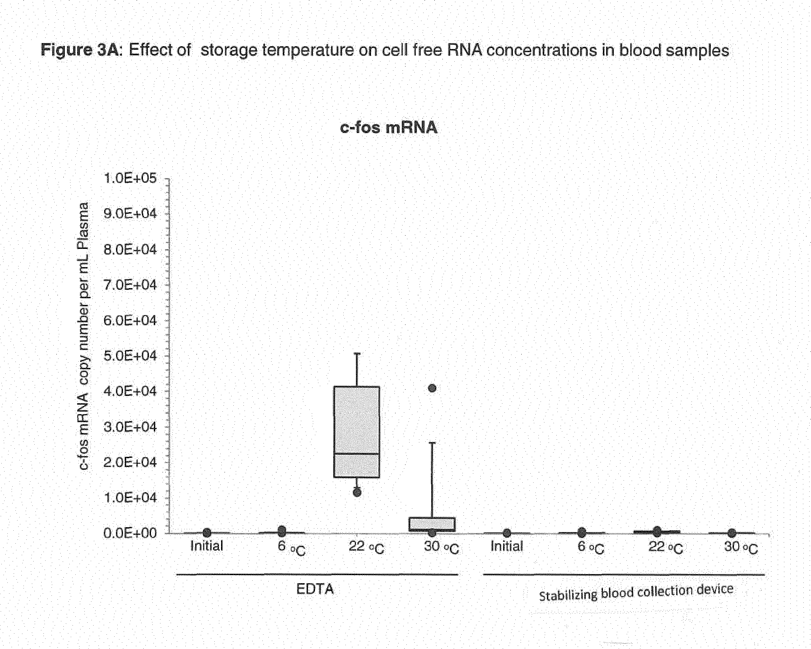

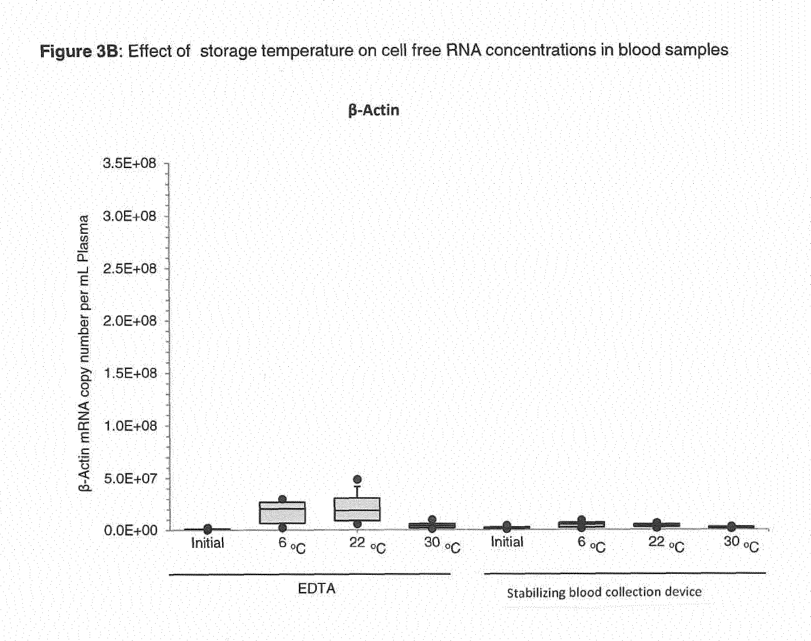

Variation in sample storage temperature is another post-phlebotomy condition that can cause undesirable changes in background RNA concentration. Here, we studied the effect of three different storage temperatures on the mRNA copy numbers of blood drawn into K₃EDTA and the stabilizing blood collection device taught herein. Following blood draw, samples were incubated at 6°C, 22°C or 30°C for 3 days. Significant increases in K₃EDTA blood sample background RNA concentrations were seen at 6°, 22°C and 30°C by day 3 (as measured by c-fos, and β-Actin markers) (Figs. 3A-3B). As shown, blood subject to the method of this invention drawn into the stabilizing blood collection device showed no significant increase in mRNA copy number at any temperature on day 3.

The stabilizing blood collection device used in the method of this invention may include imidazolidinyl urea and glycine or diazolidinyl urea and glycine. The stabilizing blood collection device may also include aurintricarboxylic acid and sodium fluoride. The stabilizing blood collection device may also include some combination of diazolidinyl urea, imidazolidinyl urea, aurintricarboxylic acid, glyceraldehyde, and sodium fluoride, and EDTA. The imidazolidinyl urea may be present in an amount of from about 100 g/l to about 1000 g/l. The imidazolidinyl urea may be present in an amount of from about 300 g/l to about 600 g/l. The diazolidinyl urea may be present in an amount of from about 50 g/l to about 800 g/l. The diazolidinyl urea may be present in an amount of from about 100 g/l to about 400 g/l. The EDTA may be present in an amount of from about 20 g/l to about 150 g/l. The EDTA may be present in an amount of from about 50 g/l to about 100 g/l. The glycine may be present in an amount of from about 10 g/l to about 150 g/l. The glycine may be present in an amount of from about 35 g/l to about 100 g/l. The glyceraldehyde may be present in an amount of from about 10 g/l to about 150 g/l. The glyceraldehyde may be present in an amount of from about 35 g/l to about 100 g/l. The aurintricarboxylic acid may be present in an amount of from about 1 g/l to about 40 g/l. The aurintricarboxylic acid may be present in an amount of from about 5 g/l to about 20 g/l. The sodium fluoride may be present in an amount of from about 0.1 g/l to about 30 g/l. The sodium fluoride may be present in an amount of from about 0.5 g/l to about 10 g/l.

For the examples below, blood donors were recruited with informed consent from Streck, Inc. in Omaha, NE. Donors were both male and female and presumed to be healthy. All draws were performed using venipuncture.

### Examples

To study the effect of storage temperature on cfRNA concentration, samples were stored at 6°, 22° and 30°C for 3 days. For each experiment, plasma was separated at various time points by centrifugation at 300 x g for 20 min followed by transferring the upper plasma layer to a new tube, and then re-centrifuging at 5,000 x g for 10 min. Total plasma cfRNA was extracted and reverse transcriptase real-time PCR (RT-qPCR) was used to quantify mRNAs for c-fos, β-actin, and18S rRNA.

To study the effect of transportation, blood was drawn from 10 donors. Blood was drawn from each donor into three 10 mL K₃EDTA tubes and three 10 mL stabilizing blood collection devices in accordance with the teachings herein. One K₃EDTA tube and one stabilizing blood collection device from each donor were processed within two hours (2h) of blood draw. Another K₃EDTA tube and stabilizing blood collection device from each donor were shipped with a temperature tracking device to a laboratory in Springfield, MA and back to Nebraska during the course of three days. The remaining K₃EDTA tube and stabilizing blood collection device from each donor were kept at 22°C for three days and processed with the returned shipped blood tubes.

### Results

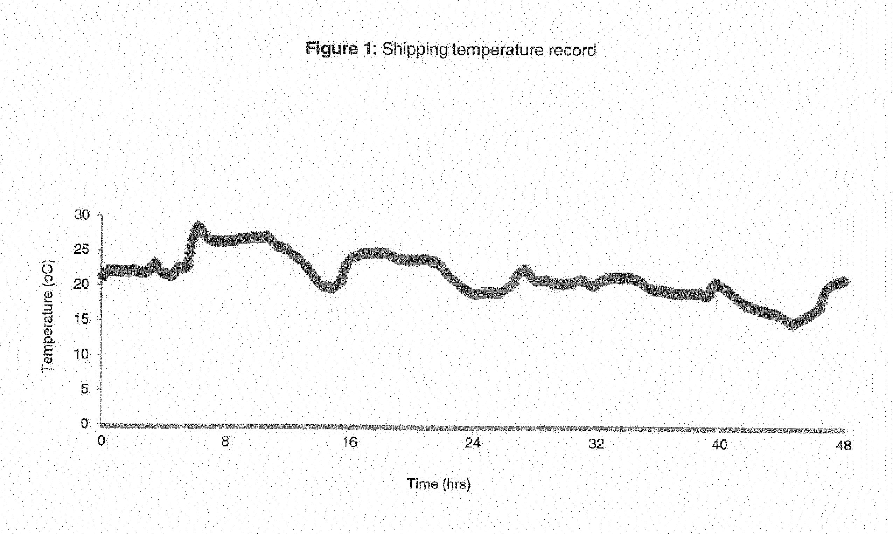

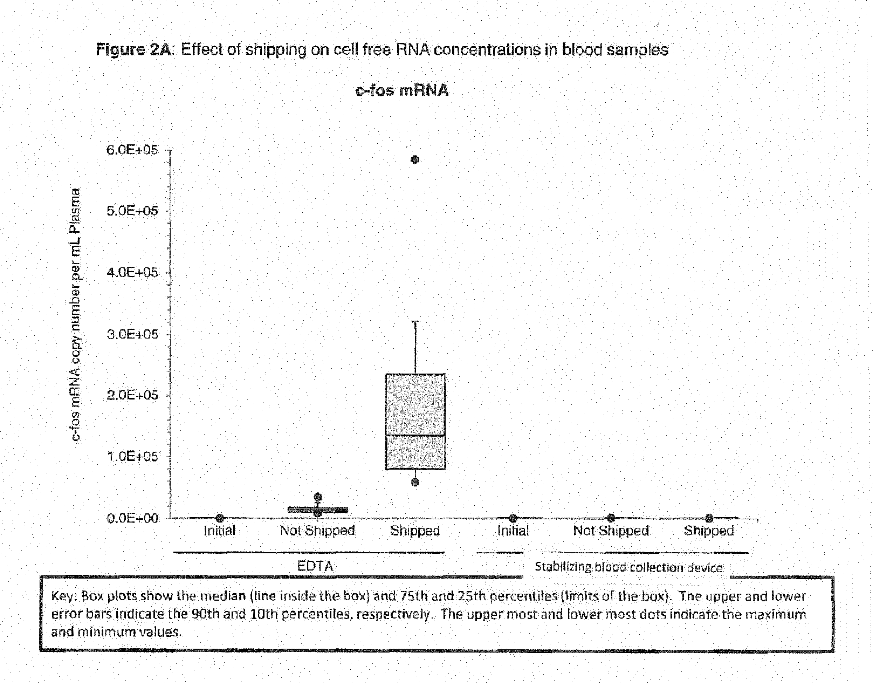

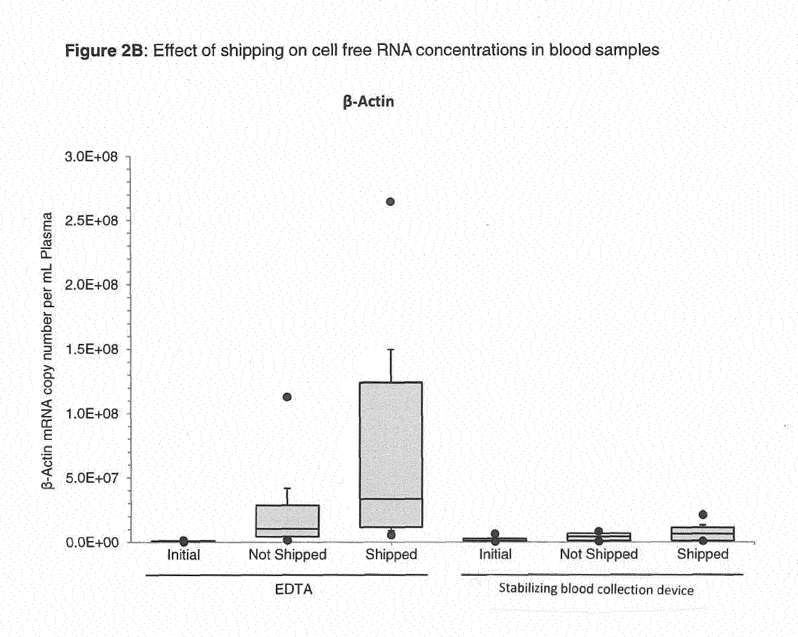

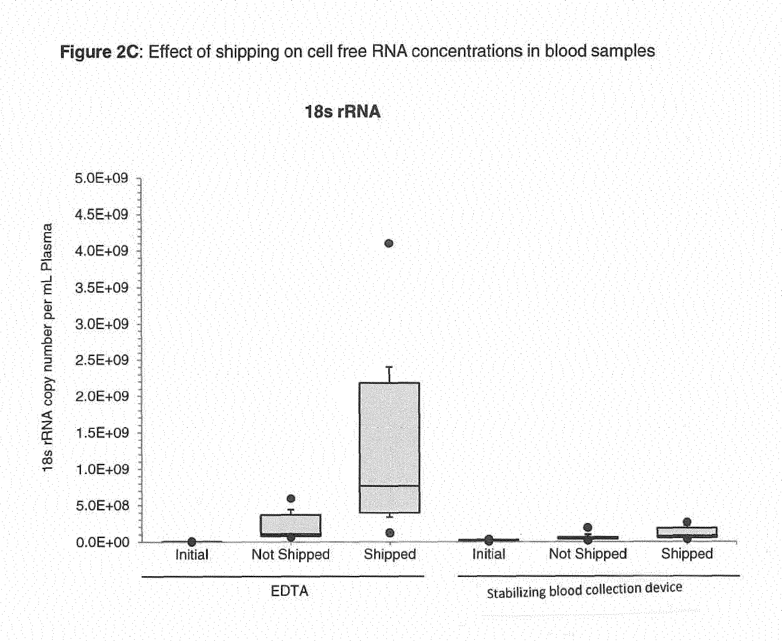

The temperature tracking device kept inside the shipping container showed a temperature range of 15° - 29°C (see Fig. 1). As demonstrated at Figs. 2A-2C, shipped blood samples drawn into K₃EDTA tubes showed a significant increase in mRNA or rRNA copy numbers for β-actin, c-fos, and 18S rRNA. In contrast, shipped blood samples drawn into stabilizing blood collection devices showed only a slight change in mRNA or rRNA copy numbers for β-actin, c-fos, and 18S rRNA. As shown in Figs. 3A-3B, blood stored in K₃EDTA tubes at 6, 22 and 30°C for 3 days showed a significant increase in mRNA copy numbers for β-actin or c-fos. Compared to K₃EDTA tubes, blood stored in stabilizing blood collection devices at 6, 22 and 30°C for 3 days showed slight to moderate increases in mRNA copy numbers for β-actin and c-fos.

The results above demonstrate that the use of the stabilizing blood collection devices taught herein have a dramatic and previously unrecognized effect on the amount of background RNA that results from shipping and storage temperatures above freezing. It can be appreciated that the stabilizing blood collection devices not only protect target nucleic acids from degradation, but also maintain the quantity of such target nucleic acids. As a result, these quantities remain substantially stable without the intrusion of background nucleic acids so that the relative quantities can be effectively utilized for measurements that may assist in disease diagnosis and tracking.

It will be appreciated that concentrates or dilutions of the amounts recited herein may be employed. In general, the relative proportions of the ingredients recited will remain the same. Thus, by way of example, if the teachings call for 30 parts by weight of a Component A, and 10 parts by weight of a Component B, the skilled artisan will recognize that such teachings also constitute a teaching of the use of Component A and Component B in a relative ratio of 3:1. Teachings of concentrations in the examples may be varied within about 25% (or higher) of the stated values and similar results are expected. Moreover, such compositions of the examples may be employed successfully in the present methods to isolate fetal nucleic acids (e.g., cell-free RNA).

It will also be appreciated that the above is by way of illustration only. Other ingredients may be employed in any of the compositions disclosed herein, as desired, to achieve the desired resulting characteristics. Examples of other ingredients that may be employed include antibiotics, anesthetics, antihistamines, preservatives, surfactants, antioxidants, unconjugated bile acids, mold inhibitors, nucleic acids, pH adjusters, osmolarity adjusters, or any combination thereof.

## Claims

1. A method for handling a biological sample to a remote site for cell-free RNA analysis, comprising the steps of:
a. drawing a blood sample directly into a stabilizing blood collection device at a blood draw site, such blood collection device including an effective amount of a stabilizing agent selected from imidazolidinyl urea and glycine and diazolidinyl urea and glycine, the sample of blood including an initial amount of background RNA and an amount of cell free RNA;
b. treating one or more components of the sample with the stabilizing agent for mitigating the propensity of background RNA within the sample to increase relative to the initial amount;
c. transporting the sample, while it remains in the blood collection device, for delivery to site at which cell-free RNA analysis is to be performed, during which transporting step the blood collection device and the sample contained therein is subjected to one or more temperatures within a range of 5 to 35 °C;
d. performing cell free RNA analysis on the sample at least 24 hours after the drawing of the sample.

2. The method of Claim 1 wherein the cell free RNA analysis is performed at least 72 hours after the drawing of the sample.

3. The method of Claim 1 or Claim 2 wherein the step of performing cell free RNA analysis on the sample includes performing transcriptase real-time PCR (RT-qPCR).

4. The method of Claim 3 wherein the step of performing cell free RNA analysis on the sample includes performing transcriptase real-time PCR (RT-qPCR) to quantify mRNAs for c-fos, β-actin, and/or 18S rRNA.

5. The method of any of the preceding claims wherein the c-fos mRNA copy number per mL of plasma within the blood sample remains substantially the same prior to transportation and post transportation.

6. The method of any of the preceding claims wherein the β-Actin mRNA copy number per mL of plasma within the blood sample remains substantially the same prior to transportation and post transportation.

7. The method of any of the preceding claims wherein the 18s rRNA copy number per mL of plasma within the blood sample remains substantially the same prior to transportation and post transportation.

8. The method of any of the preceding claims wherein the amount of background RNA in the blood sample remains substantially the same prior to transportation and post transportation.

## Patentansprüche

1. Verfahren zum Handhaben einer biologischen Probe an einen entfernten Standort zur zellenfreien RNA-Analyse, welches die folgenden Schritte umfasst:
a. Entnehmen einer Blutprobe direkt in ein stabilisierendes Blutsammelgerät an einem Blutentnahmestandort, wobei ein solches Blutsammelgerät eine effektive Menge eines stabilisierenden Mittels enthält, das ausgewählt ist aus Imidazolidinylharnstoff und Glycin sowie Diazolidinylharnstoff und Glycin, wobei die Blutprobe eine anfängliche Menge von Hintergrund-RNA und eine Menge an zellenfreier RNA enthält;
b. Behandeln einer oder mehrerer Komponenten der Probe mit dem stabilisierenden Mittel zum Abschwächen der Neigung von Hintergrund-RNA in der Probe im Vergleich zu der anfänglichen Menge anzuwachsen;
c. Transportieren der Probe, während sie in dem Blutsammelgerät bleibt, zur Lieferung an einen Standort, an dem eine zellenfreie RNA-Analyse auszuführen ist, wobei während des Transportschritts das Blutsammelgerät und die darin enthaltene Probe einer oder mehreren Temperaturen unterworfen werden, die in einem Wertebereich von 5 bis 35 °C liegen;
d. Ausführen einer zellenfreien RNA-Analyse bezüglich der Probe wenigstens 24 Stunden nach dem Entnehmen der Probe.

2. Verfahren nach Anspruch 1, wobei die zellenfreie RNA-Analyse wenigstens 72 Stunden nach dem Entnehmen der Probe ausgeführt wird.

3. Verfahren nach Anspruch 1 oder Anspruch 2, wobei der Schritt zum Ausführen einer zellenfreien RNA-Analyse bezüglich der Probe ein Ausführen von Transkriptase-Echtzeit-PCR (RT-qPCR) enthält.

4. Verfahren nach Anspruch 3, wobei der Schritt zum Ausführen einer zellenfreien RNA-Analyse bezüglich der Probe ein Ausführen einer Transkriptase-Echtzeit-PCR (RT-qPCR) enthält, um mRNAs für c-fos, β-Actin und/oder 18S rRNA zu quantifizieren.

5. Verfahren nach einem der vorhergehenden Ansprüche, wobei die c-fos-mRNA-Kopienanzahl pro mL von Plasma in der Blutprobe vor dem Transport und nach dem Transport im Wesentlichen dieselbe bleibt.

6. Verfahren nach einem der vorhergehenden Ansprüche, wobei die β-ActinmRNA-Kopienanzahl pro mL Plasma in der Blutprobe vor dem Transport und nach dem Transport im Wesentlichen dieselbe bleibt.

7. Verfahren nach einem der vorhergehenden Ansprüche, wobei die 18s-rRNA-Kopienanzahl pro mL Plasma in der Blutprobe vor dem Transport und nach dem Transport im Wesentlichen dieselbe bleibt.

8. Verfahren nach einem der vorhergehenden Ansprüche, wobei die Menge an Hintergrund-RNA in der Blutprobe vor dem Transport und nach dem Transport im Wesentlichen dieselbe bleibt.

## Revendications

1. Un procédé pour manipuler un échantillon biologique jusqu'à un site distant en vue d'une analyse d'ARN acellulaire, comportant les étapes consistant à :
a. prélever un échantillon de sang directement dans un dispositif de prélèvement sanguin et de stabilisation sur un site de prélèvement sanguin, un tel dispositif de prélèvement sanguin incluant une quantité efficace d'un agent de stabilisation choisi parmi l'imidazolidinyl urée et la glycine et la diazolidinyl urée et la glycine, l'échantillon de sang incluant une quantité initiale d'ARN de fond et une quantité d'ARN acellulaire,
b. traiter un ou plusieurs composants de l'échantillon avec l'agent de stabilisation pour atténuer la propension de l'ARN de fond dans l'échantillon à augmenter par rapport à la quantité initiale,
c. transporter l'échantillon, alors qu'il reste dans le dispositif de prélèvement sanguin, pour une livraison au site où l'analyse d'ARN acellulaire doit être effectuée, étape de transport pendant laquelle le dispositif de prélèvement sanguin et l'échantillon contenu dans celui-ci sont soumis à une ou plusieurs températures sur une plage de 5 à 35 °C,
d. effectuer une analyse d'ARN acellulaire sur l'échantillon au moins 24 heures après le prélèvement de l'échantillon.

2. Le procédé selon la revendication 1, dans lequel l'analyse d'ARN acellulaire est réalisée au moins 72 heures après le prélèvement de l'échantillon.

3. Le procédé selon la revendication 1 ou la revendication 2, dans lequel l'étape de réalisation de l'analyse d'ARN acellulaire sur l'échantillon inclut la réalisation d'une PCR en temps réel après transcription inverse (RT-qPCR).

4. Le procédé selon la revendication 3, dans lequel l'étape de réalisation de l'analyse d'ARN acellulaire sur l'échantillon inclut de réaliser une PCR en temps réel après transcription inverse (RT-qPCR) pour quantifier les ARNm pour la c-fos, la β-actine et/ou l'ARNr 18S.

5. Le procédé selon l'une quelconque des revendications précédentes, dans lequel le nombre de copies d'ARNm pour la c-fos par mL de plasma dans l'échantillon de sang reste sensiblement le même avant et après le transport.

6. Le procédé selon l'une quelconque des revendications précédentes, dans lequel le nombre de copies d'ARNm pour la β-actine par mL de plasma dans l'échantillon de sang reste sensiblement le même avant et après le transport.

7. Le procédé selon l'une quelconque des revendications précédentes, dans lequel le nombre de copies d'ARNr 18S par mL de plasma dans l'échantillon de sang reste sensiblement le même avant et après le transport.

8. Le procédé selon l'une quelconque des revendications précédentes, dans lequel la quantité d'ARN de fond dans l'échantillon de sang reste sensiblement la même avant et après le transport.
